# EUROPEAN PATENT APPLICATION

(11) **EP 2 857 521 A1**
(43) Date of publication of application: **08.04.2015**
(21) Application number: 13796783.2
(22) Date of filing: 30.05.2013
(51) Int. Cl.: C12Q 1/68, C12N 15/09, G01N 33/48

(54) **METHOD FOR ASSESSING ENDOMETRIOSIS**

(30) Priority: 31.05.2012 JP 2012124595
(71) Applicant: Takeda Pharmaceutical Company Limited, Osaka-shi, Osaka 541-0045 (JP)
(72) Inventor: UCHIYAMA, Hidefumi, Fujisawa Kanagawa 251-0012 (JP)
(74) Representative: Heubeck, Christian
(86) International application number: PCT/JP2013/003428
(87) International publication number: WO 2013/179672

(57) **Abstract**

The present invention provides a diagnostic agent for endometriosis for measuring the concentration of at least one miRNA selected from the group consisting of hsa-miR-708, hsa-miR-127-3p and hsa-miR-518d-3p in a sample derived from the blood of a subject, the agent containing an amplification primer for the miRNA as a main component. The present invention also provides a method of detecting endometriosis, including a step of measuring the concentration of at least one miRNA selected from the group consisting of hsa-miR-708, hsa-miR-127-3p and hsa-miR-518d-3p in a sample derived from the blood of the subject. The diagnostic agent for endometriosis of the present invention and the diagnostic method using the agent are simple and low invasive and have high sensitivity and specificity.

SEQUENCE LISTING

<110>  Takeda Pharmaceutical Company limited

<120>  Diagnosis of Endometriosis

<130>  PT38-9001WO

<150>  JP 2012-124595
<151>  2012-05-31

<160>  3

<170>  PatentIn version 3.1

<210>  1
<211>  23
<212>  RNA
<213>  Homo sapiens

<400>  1
aaggagcuua caaucuagcu ggg                                               23


<210>  2
<211>  22
<212>  RNA
<213>  Homo sapiens

<400>  2
ucggauccgu cugagcuugg cu                                                22


<210>  3
<211>  21
<212>  RNA
<213>  Homo sapiens

<400>  3
caaagcgcuu cccuuuggag c                                                 21

## Description

### Related Application

The present application claims a priority right based on Japanese Patent Application No. 2012-124595 (filed May 31, 2012), the content of which is incorporated herein by reference.

### Technical Field

The present invention relates to a novel endometriosis diagnostic marker and diagnosis of endometriosis using the marker.

### Background of the Invention

Endometriosis is presently diagnosed by transvaginal ultrasonography and magnetic resonance imaging and confirmed by laparoscopic inspection. As a diagnostic marker for endometriosis, a blood-protein marker such as CA125 is known.

In addition to CA125, serum CA19-9 has been studied on availability for a diagnostic marker (FERTILITY AND STERILITY 78 (2002) 733-739: Non Patent Literature 1). Furthermore, a diagnostic method using CA-125 in combination with various inflammation markers (all are protein markers) has been proposed (FERTILITY AND STERILITY 89 (2008) 1073-1081: Non Patent Literature 2).

Moreover, miRNA expression analysis performed in the endometrial membrane of endometriosis patients has been reported (Mol Endocrinol 25 (2011) 821-832: Non Patent Literature 3). In the Literature, it is described that expression of 10 miRNAs increases; whereas expression of 12 miRNAs decreases in the endometrial tissue of patients. However, the literature is silent about expression of these miRNAs in the blood.

The "miRNA (microRNA)" refers to a single-stranded RNA molecule consisting of 19 to 23 bases and endogenously present in a living body. Up to present, 700 types or more of human miRNAs have been identified and found to act on mRNA (messenger RNA) of a target gene in a sequence dependent manner to control gene expression; however, the detailed mechanism of the action and the physiological role of the human miRNAs have not yet been sufficiently elucidated.

Literatures cited for reference herein are as follows. The contents of these Literatures are incorporated herein in their entirety by reference.

### Citation List

### Non Patent Literature

Non Patent Literature 1: FERTILITY AND STERILITY 78 (2002) 733-739
Non Patent Literature 2: FERTILITY AND STERILITY 89 (2008) 1073-1081
Non Patent Literature 3: Mol Endocrinol 25 (2011) 821-832

### Summary of Invention

### Technical Problem

An object of the present invention is to provide an endometriosis diagnostic marker in a blood-derived sample, which is simpler and less invasive and has higher sensitivity and specificity than the markers used in conventional methods. Another object of the present invention is to provide endometriosis diagnostic markers in a blood-derived sample which are easily used in combination, and provide a diagnostic method using the markers.

### Solution to Problem

The present inventors checked expression of about 600 types of miRNAs in samples derived from the blood of endometriosis patients. As a result, they found that expression of three types of miRNAs: hsa-miR-708, hsa-miR-127-3p and hsa-miR-518d-3p increased. Based on the finding, the present inventors conducted further intensive studies and accomplished the present invention.

More specifically, the present invention provides
[1] A diagnostic agent for endometriosis for measuring the concentration of at least one miRNA selected from the group consisting of hsa-miR-708, hsa-miR-127-3p and hsa-miR-518d-3p in a sample derived from the blood of a subject, the agent comprising an amplification primer for the miRNA as a main component;
[2] A kit for diagnosing endometriosis comprising the diagnostic agent according to [1];
[3] The diagnostic agent for endometriosis according to [1], in which the miRNA is hsa-miR-708;
[4] The diagnostic agent for endometriosis according to [1], in which the miRNA is hsa-miR-127-3p;
[5] The diagnostic agent for endometriosis according to [1], in which the miRNA is hsa-miR-518d-3p;
[6] The diagnostic agent for endometriosis according to any one of [1] and [3] to [5], wherein the sample derived from the blood is blood plasma;
[7] A method of detecting endometriosis, comprising a step of measuring the concentration of at least one miRNA selected from the group consisting of hsa-miR-708, hsa-miR-127-3p and hsa-miR-518d-3p in a sample derived from the blood of a subject;
[8] The method according to [7], comprising a step of determining whether the concentration of the miRNA is higher than the concentration of the miRNA in a normal control;
[9] The method according to [7] or [8], comprising a step of determining whether the concentration of the miRNA is a cutoff value or more;
[10] The method according to any one of [7] to [9], wherein the miRNA is hsa-miR-708;
[11] The method according to any one of [7] to [9], wherein the miRNA is hsa-miR-127-3p;
[12] The method according to any one of [7] to [9], wherein the miRNA is hsa-miR-518d-3p;
[13] The method according to any one of [7] to [12], wherein the sample derived from the blood is blood plasma;
[14] A method for diagnosing endometriosis in a subject, comprising a step of measuring the concentration of at least one miRNA selected from the group consisting of hsa-miR-708, hsa-miR-127-3p and hsa-miR-518d-3p in a sample derived from the blood of the subject;
[15] The method according to [14], comprising a step of determining whether the concentration of the miRNA is higher than the concentration of the miRNA in a normal control; and
[16] The method according to [14] or [15], comprising a step of determining whether the concentration of the miRNA is a cutoff value or more.

### Advantageous Effects of Invention

The diagnostic agent and method for endometriosis according to the present invention are simpler and less invasive and have higher sensitivity and specificity than a conventional method (transvaginal ultrasonography and magnetic resonance imaging, laparoscopic inspection, blood-protein marker, etc.). Furthermore, in the diagnostic agent and method for endometriosis of the present invention, measurement can be easily made in combination with plural markers. Owing to the combination of plural markers, endometriosis can be diagnosed with a high sensitivity and specificity.

### Brief Description of Drawings

[Figure 1] Figure 1 shows distribution of expression of three-type miRNAs: hsa-miR-708, hsa-miR-127-3p and hsa-miR-518d-3p in two groups: a patient group and a control group.
[Figure 2] Figure 2 shows detectability of endometriosis by a single miRNA, evaluated by ROC analysis, in which the vertical axis represents sensitivity; whereas the transverse axis represents a false-positive rate (1-Specificity).
[Figure 3] Figure 3 shows detectability evaluation by use of a prediction model using plural miRNAs.

### Description of Embodiments

The present invention provides a diagnostic agent for endometriosis (sometimes referred to as "the diagnostic agent of the present invention" in this specification) for measuring the concentration of at least one miRNA (sometimes referred to as "miRNA of the present invention" in this specification) selected from the group consisting of hsa-miR-708, hsa-miR-127-3p and hsa-miR-518d-3p in a sample derived from the blood of a subject, the agent containing an amplification primer for the miRNA as a main component.

The subject in this specification refers to a human having endometriosis or suspected to have endometriosis. Examples of the sample derived from the blood of a subject include whole blood, blood serum and blood plasma. The sample is preferably blood serum or blood plasma and further preferably blood plasma. Note that it is generally believed that the serum miRNA concentration correlates with the plasma miRNA concentration (for example, see Proc. Natl. Acad. Sci. USA, 105 (2008) 10513-10518).

In the present invention, miRNAs which have been found to be overexpressed in a sample derived from the blood of an endometriosis patient, are three types of miRNAs: hsa-miR-708, hsa-miR-127-3p and hsa-miR-518d-3p. The base sequences of these miRNAs are as follows and can be found in miRBase (http: //www.mirbase.org/index.shtml).
hsa-miR-708 (aaggagcuuacaaucuagcuggg) SEQ ID No:1
hsa-miR-127-3p (ucggauccgucugagcuuggcu) SEQ ID No:2
hsa-miR-518d-3p (caaagcgcuucccuuuggagc) SEQ ID No:3

The diagnostic agent for endometriosis of the present invention contains a primer (sometimes referred to as "the amplification primer of the present invention" in this specification) capable of amplifying at least one of these three types of miRNAs, as a main component. The concentration of the miRNA of the present invention in a blood-derived sample can be measured by amplifying the miRNA of the present invention by a polymerase chain reaction (PCR), etc. using these amplification primers, with the result that endometriosis can be simply and quickly examined.

The amplification primer of the present invention is a single-stranded oligonucleotide and preferably DNA. The amplification primer includes a forward primer and a reverse primer and they are used in combination. The forward primer typically has a homologous sequence to the sequence consisting of 6 to 10 bases, which are present on the 5' side of the center nucleic acid of a miRNA sequence (however, if the primer is DNA, T is contained in place of U); whereas, the reverse primer has a complementary sequence to the sequence consisting of 6 to 10 bases, which are present on the 3' side of the center nucleic acid of a miRNA sequence.

The primer may optionally have an additional sequence (for example, a sequence encoding His-tag and restriction enzyme recognition sites) useful for recognizing, purifying, and sub-cloning of an amplified product, on the side opposite to the direction of extension. Such an additional sequence preferably has a length of 1 to 15 bases. The sequences of the amplification primers are selected such that the primers each have high specificity, do not form a secondary structure within their molecules and are not mutually hybridized. Each amplification primer has a length of preferably 12 to 30 nucleotides and more preferably 15 to 25 nucleotides.

To simply measure the amount of PCR product, one or both of the forward primer and the reverse primer are preferably tagged with a fluorescent label. Examples of the fluorescent label include a fluorescent dye, a quenching substance, a donor pigment and an acceptor pigment.

The amplification primer of the present invention can be chemically synthesized by a general DNA synthesis apparatus (for example, Model 394, manufactured by Applied Biosystems); however, another method well known in the art may be employed for synthesis.

In another aspect, the present invention provides a kit for diagnosing endometriosis. The kit may contain not only the diagnostic agent of the present invention but also e.g., pretreatment reagents for a sample, reagents and enzyme for PCR, a buffer solution, a container and instruction for use.

In yet another aspect, the present invention provides a method for detecting endometriosis (sometimes referred to as "the method of the present invention" herein) including a step of measuring the concentration of at least one miRNA selected from the group consisting of hsa-miR-708, hsa-miR-127-3p and hsa-miR-518d-3p, in a sample derived from the blood of a subject.

To measure the concentration of miRNA in a sample, RNA is extracted from the sample, template cDNA is prepared and PCR is performed using the template cDNA as a template and the amplification primer of the present invention. In this manner, at least one miRNA selected from the group consisting of hsa-miR-708, hsa-miR-127-3p and hsa-miR-518d-3p contained in the sample is amplified.

A method of extracting RNA from a sample is well known in the art. For example, guanidine-isothiocyanate and phenol chloroform extraction can be used. Alternatively, a commercially available RNA extraction reagent can be used. Subsequently, the obtained total RNA is fractionated to obtain miRNA. For example, RNA is extracted from a sample by use of Trizol LS reagent (Life Technologies Inc.) and fractionated by use of a filter cartridge of mirVana miRNA isolation kit (Life Technologies Inc.) to obtain an RNA fraction.

A method of synthesizing cDNA using RNA as a template is also well known in the art. In the method, cDNA can be synthesized using a commercially available random primer or Stem-loop reverse transcription primers as the primer in the presence of dNTPs and a reverse transcriptase.

Subsequently, PCR is performed using DNA or cDNA thus prepared as a template and the amplification primer of the present invention. Preferably, miRNA is quantified using real time PCR amplification method. In a particularly preferable aspect, the concentration of miRNA in a blood-derived sample can be measured by a quantitative RT-PCR using a TaqMan (trade name) probe.

To correct variation of measurement values in measuring miRNA concentration, an appropriate internal standard is preferably included. Examples of the internal standard include RNAs, which are known not to change in expression level, such as miRNA, snRNA and mRNA. Specific examples thereof include β-actin, glyceraldehyde β-phosphate dehydrogenase and ribosomal protein P1. In the case of measuring a great number (for example 200 or more) of miRNAs at the same time, medium values of expression levels of all the miRNAs can be used as the internal standard.

The concentration of the miRNA of the present invention may be measured by a hybridization method using a microarray on which a probe having a homologous sequence or a complementary sequence to the sequence of a part of hsa-miR-708, hsa-miR-127-3p or hsa-miR-518d-3p is immobilized. Alternatively, the concentration of the miRNA can be directly measured by use of a next-generation sequencing.

It is determined whether the concentration of the miRNA of the present invention obtained as mentioned above in a sample derived from the blood of a subject is higher than the concentration of the miRNA of the present invention in a normal control. If the concentration of the miRNA of the present invention in the subject's sample as mentioned above is higher than that of a normal control, it is determined that the subject has endometriosis or suspected to have endometriosis. The "normal control" refers to a sample derived from the blood of a human who was diagnosed not to have endometriosis. It is preferable that concentrations of each miRNA in plural normal controls were measured in advance to obtain an average expression level of the control group.

In a preferable aspect, the method of the present invention includes a step of determining whether a prediction model function value, which is constructed using the concentration of miRNA in a sample derived from the blood of a subject, is a cutoff value or more. The cutoff value refers to a value, which is set in order to determine and diagnose a disease. The cutoff value of the present invention is a value of a prediction model function, which is constructed using the concentration of the miRNA of the present invention in a sample derived from the blood of a subject, and set in order to find whether or not the subject is suspected to have endometriosis. If the concentration of the miRNA of the present invention in the subject's sample as mentioned above is a cutoff value or more, it is determined that the subject has endometriosis or is suspected to have endometriosis. How to set such a cutoff value is not particularly defined; however, the cutoff value can be obtained by constructing a prediction model function taking a value between 0 and 1 by logistic regression and subjecting a predetermined subject group and a control group to ROC curve analysis.

In a specific aspect, the following values can be used as the cutoff value:
When three miRNAs: has-miR-708, has-miR-127-3p and has-miR-518d-3p, are used as miRNA in the sample, a value of 0.1 or more and 0.9 or less, preferably 0.31 or more and 0.68 or less and more preferably 0.40 or more and 0.59 or less can be used as the cutoff value.
When two miRNAs: has-miR-708 and has-miR-518d-3p, are used as miRNA in the sample, a value of 0.1 or more and 0.9 or less, preferably 0.34 or more and 0.66 or less and more preferably 0.38 or more and 0.54 or less can be used, as the cutoff value.
When has-miR-708 is used as miRNA in the sample, a value of 0.1 or more and 0.9 or less, preferably 0.42 or more and 0.60 or less and more preferably 0.46 or more and 0.58 or less can be used as the cutoff value.
When has-miR-127-3p is used as miRNA in the sample, a value of 0.1 or more and 0.9 or less, preferably 0.44 or more and 0.55 or less and more preferably 0.49 or more and 0.55 or less can be used as the cutoff value.
When has-miR-518d-3p is used as miRNA in the sample, a value of 0.1 or more and 0.9 or less, preferably 0.44 or more and 0.56 or less and more preferably 0.47 or more and 0.53 or less can be used as the cutoff value.

Furthermore, the method of the present invention can be applied to the case of measuring the concentration of the miRNA of the present invention in a sample derived from the blood only taken from a single subject once in a certain time point. Since the concentration of the miRNA of the present invention increases with the passage of time from the onset of endometriosis, the method of the present invention can be applied to the case of measuring the concentration of the miRNA of the present invention in samples taken from a single subject with the passage of time. This case is preferred since endometriosis can be detected without comparing with the concentration of the miRNA in a normal control. In this case, a sample is taken, for example, at an interval of e.g., once per week during the period from two weeks after a first medical examination to full recovery. More specifically, the diagnostic method of the present invention includes a method of monitoring progression of a disease and a method of monitoring therapeutic process.

The contents of the all patent literatures and reference literatures expressly cited in the specification are incorporated in their entirety in the specification by reference. The aspect expressed by the phrase "...comprising" used in the specification includes the aspect expressed by the phrase "... essentially consisting of" and an aspect expressed by the phrase "...consisting of".

Now, the present invention will be more specifically described by way of Examples; however, the present invention is not limited by these Examples.

### Examples

### (Serum samples from patients and control subjects)

Serum samples of endometriosis patients (20 cases) diagnosed by a transvaginal hysteroscope and control subjects (20 cases, 9 cases of them had benign gynecological diseases except endometriosis, 11 cases were healthy persons) having the corresponding age composition to the patient group, were purchased from Cureline Inc. (South San Francisco, California, U.S.A.). The serum samples were stored at -80°C until use. Clinical information of the subjects are shown in Table 1.

**[Table 1]**

| Clinical information of subject | | | | | | | | | |
|---|---|---|---|---|---|---|---|---|---|
| subject ID | Height (cm) | Weight (kg) | Estradiol (pM) | Progesteron (nM) | FSH (mMU/mL) | Days after last period | Age | Diagnosis | Smocking (year) |
| EM01 | 156 | 70 | 801.03 | 3.95 | 6.87 | 6 | 33 | stage II | |
| EM02 | 159 | 74 | 584.48 | 6.25 | 2.57 | 6 | 33 | stage II | |
| EM03 | 160 | 79 | 235.00 | 1.26 | 4.58 | 5 | 32 | stage II | |
| EM04 | 174 | 55 | 1025.40 | 5.26 | 60.20 | 27 | 34 | stage I | |
| EM05 | 170 | 54 | 2146.10 | 5.92 | 8.64 | 9 | 32 | stage II | |
| EM06 | 170 | 65 | 600.00 | 2.40 | 9.70 | 7 | 54 | Genital endometriosis, adenomyosis | |
| EM07 | 170 | 72 | 60030 | 2.80 | 7.60 | 3 | 39 | Endometious ovarian cyst | |
| EM08 | 164 | 67 | 350.40 | 1.20 | 352.40 | 14 | 39 | Adenomyosis | 5 |
| EM09 | 163 | 63 | 420.50 | 2.60 | 7.90 | 21 | 39 | Genita: endometriosis, adenomyosis | |
| EM10 | 167 | 58 | 350.80 | 2.10 | 3.70 | 5 | 44 | Genital endometriosis | |
| EM17 | 158 | 50 | 250.30 | 1.20 | 18.30 | 5 | 39 | Cervical endometrisis | |
| EM12 | 170 | 62 | 350.40 | 1.80 | 6.50 | 4 | 45 | Genital endometriosis, adenomyosis | |
| EM13 | 167 | 63 | 550.40 | 7.60 | 8.70 | 4 | 44 | Genital endometriosis, adenomyosis | |
| EM14 | 170 | 64 | 78.60 | 2.70 | 5.80 | 7 | 42 | Genital endometriosis, adenomyosis | 10 |
| EM15 | 170 | 60 | 227.96 | 1.15 | 5.69 | 13 | 44 | Disseminated genital endomatriosis | |
| EM16 | 165 | 54 | 258.70 | 1.40 | 5.50 | 3 | 42 | Genital endometriosis, adenomyosis | 5 |
| EM17 | 165 | 67 | 490.00 | | 4.55 | 3 | 43 | | |
| EM18 | 170 | 70 | 540.00 | | 10.70 | 4 | 42 | | |
| EM19 | 164 | 65 | 660.00 | | 8.40 | 6 | 46 | | |
| EM20 | 166 | 59 | 408.50 | | 5.58 | 4 | 39 | | |
| EC01 | 170 | 78 | 206.82 | 2.56 | 6.30 | 2 | 33 | - | |
| EC02 | 176 | 58 | 301.39 | 2.76 | 9.10 | 19 | 32 | - | |
| EC03 | 176 | 90 | 300.25 | 7.08 | 5.50 | 2 | 33 | - | |
| EC04 | 168 | 88 | 205.30 | 6.08 | 25.40 | 15 | 30 | - | |
| EC05 | 158 | 58 | 270.80 | 4.72 | 25.40 | 7 | 34 | - | |
| EC06 | 163 | 55 | 350.40 | 2.30 | 4.80 | 5 | 54 | Healthy | |
| EC07 | 156 | 48 | 650.30 | 10.30 | 6.50 | 8 | 54 | ovarian polycystosis | |
| EC08 | 158 | 47 | 1020.10 | 2.40 | 9.30 | 36 | 39 | ovarian polycystosis | |
| EC09 | 160 | 65 | 52.40 | 1.70 | 4.60 | 5 | 45 | endometrial polyp | |
| EC10 | 165 | 49 | 273.40 | 1.80 | 2.60 | 3 | 39 | Healthy | |
| EC11 | 158 | 55 | 170.80 | 2.40 | 463.80 | 13 | 45 | cervix erosion | |
| EC12 | 162 | 54 | 208.40 | 1.60 | 5.30 | 6 | 41 | Healthy | |
| EC13 | 165 | 62 | 304.80 | 1.20 | 6.40 | 26 | 39 | cervix erosion | |
| EC14 | 168 | 54 | 228.40 | 1.40 | 2.80 | 3 | 39 | ovarian polycystosis | |
| EC15 | 170 | 58 | 320.80 | 1.40 | 5.80 | 3 | 39 | Healthy | |
| EC16 | 169 | 65 | 540.30 | 1.60 | 4.70 | 6 | 45 | uterine myoma. mixed form | |
| EC17 | 164 | 58 | 205.40 | 1.80 | 4.70 | 6 | 44 | endometrial polyp | |
| EC18 | 168 | 51 | 159.40 | 1.40 | 2.60 | 5 | 39 | Healthy | |
| EC19 | 158 | 67 | 350.80 | 1.70 | 4.20 | 10 | 45 | uteral myoma, subserosal form | |
| EC20 | 165 | 52 | 174.80 | 1.80 | 5.72 | 4 | 39 | Healthy | |

### (RNA extraction from serum samples)

RNA was extracted from serum samples by use of Trizol LS reagent and mirVana miRNA isolation kit (both manufactured by Life Technologies Inc.). Specific procedure was as follows. To a serum sample (1 mL), 1 mL of sterilized water was added. Further to the mixture, 6 mL of Trizol LS reagent was added and mixed. The solution mixture was allowed to stand still at room temperature for 5 minutes, and then 1.6 mL of chloroform was added thereto and vigorously stirred by hand. After stirring, the solution was allowed to stand still at room temperature for 10 minutes, and centrifugally separated (4°C, 12,000 × g, 10 minutes) to obtain an organic solvent layer and a water layer. From the obtained water layer, an aliquot (4 mL) was taken. To the aliquoted water layer, 5 mL of 99.5% ethanol was added and homogeneously stirred. The stirred solution was added to a filter cartridge of the mirVana miRNA isolation kit and fractionated in accordance with the protocol attached to the kit to obtain an RNA fraction. The RNA fraction was taken, lyophilized and resuspended with 10 µL of sterilized water to obtain a concentrated RNA solution. The concentration of miRNA contained in the obtained concentrated RNA solution was measured by use of Agilent 2100 bioanalyzer and Small RNA kit (Agilent Technologies) and represented in terms of the concentration of RNA having 10 to 40 base length contained in the concentrated RNA solution.

### (Quantitative analysis of miRNA)

From the above concentrated RNA solution, a cDNA-containing solution was obtained by use of Megaplex RT primers (pool A or B v.2.0) and TaqMan™ MicroRNA Reverse Transcription Kit. Thereafter, cDNA contained in the obtained solution was amplified by use of Megaplex PreAmp primers (pool A or B v.2.0) and TaqMan™ PreAmp Master Mix. The amplified cDNA was mixed with TaqMan Universal PCR Master Mix (No AmpErase UNG). The obtained sample was loaded to TaqMan™ Human MicroRNA Array v2.0 and subjected to quantitative PCR analysis by use of 7900HT Fast Real-Time PCR system. Note that each operation described in this section was performed in accordance with the protocol attached to the kit and system.

### (Data analysis)

In analyzing data, RQ Manager 1.2 software (Life Technologies) was used. A C_{T} (Threshold cycle) value was calculated from an amplification curve in accordance with the method reported by Liang et al. (Liang, Y. et al., BMC Genomics, 8, 166-, 2007), and the threshold was set at 0.2. If miRNA was not amplified in 80% or more of the total 40 specimens, the miRNA was eliminated from the analysis. The median value of C_{T} values of the remaining miRNAs was calculated for each sample. Then, a value (ΔC_{T} values) was obtained by subtracting the medium value from each of the C_{T} values to normalize the expression difference between the specimens. To identify miRNA whose expression level significantly differs between the patient group and the control group, Welch's t-test was carried out. Plural miRNAs identified by using logistic regression analysis, were used in combination to construct and evaluate a diagnosis model. Furthermore, diagnostic ability by each of the miRNAs or a combination of miRNAs was evaluated by ROC (Receiver operating characteristic) analysis. For these analyses, MATLAB software (The Mathworks Inc.) was used.

### (Results)

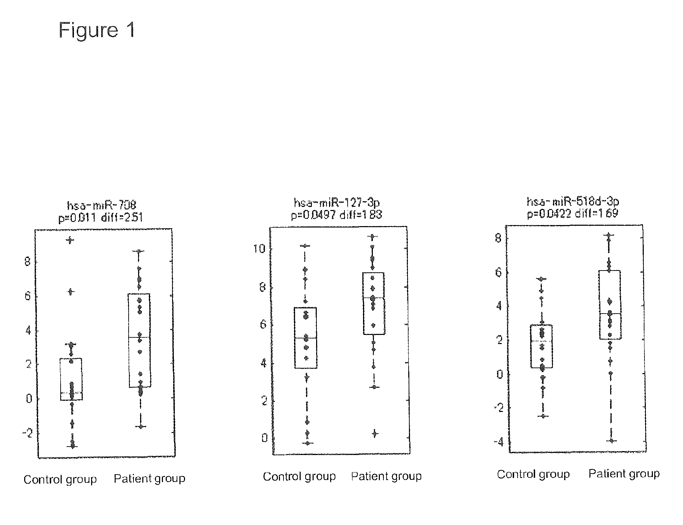

Figure 1 shows distribution of expression of three-type miRNAs in two groups: a patient group and a control group. The vertical axis represents the relative expression level of each of the miRNAs by a logarithm to base 2. The expression level of each subject is plotted by a blue dot and the median value of expression level in each group is indicated by a red line. The upper limit and lower limit of each box indicate 25 percentile and 75 percentile, respectively. Both ends of each whisker represent the upper limit and lower limit of the values from which outliers are eliminated. Red crosses represent outliners. The P value obtained as a result of the Welch's t-test and the difference in average expression level (logarithm using 2 as the base) between both groups are indicated under the name of miRNA in each Figure.

The expression profile of miRNAs in the sera of the endometriosis patient group (20 cases) was compared with that of the control subject group (20 cases). As a result, it was found that expression levels of three-type miRNAs: hsa-miR-708, hsa-miR-127-3p, and hsa-miR-518d-3p, significantly increased in the patient group (Figure 1). From this, it was clearly demonstrated that the patient group can be satisfactorily detected by measuring expression levels of these three miRNAs in the serum.

Table 2 shows the sequences of these miRNAs and the expression-level increase rates (Fold Change) of these miRNAs in the patient group compared to the control group, calculated from average values of expression levels (logarithm using 2 as the base) of both groups.

**[Table 2]**

| Name of miRNA | Base sequence | Expression increase rate in patient group | Sequence No. |
|---|---|---|---|
| hsa-miR-708 | aaggagcuuacaaucuagcuggg | 5.71 | 1 |
| hsa-miR-127-3p | ucggauccgucugagcuuggcu | 3.56 | 2 |
| hsa-miR-518d-3p | caaagcgcuucccuuuggagc | 3.24 | 3 |

In the case where these upregulated three miRNAs were used for detection of endometriosis, the detectability was evaluated by ROC analysis.

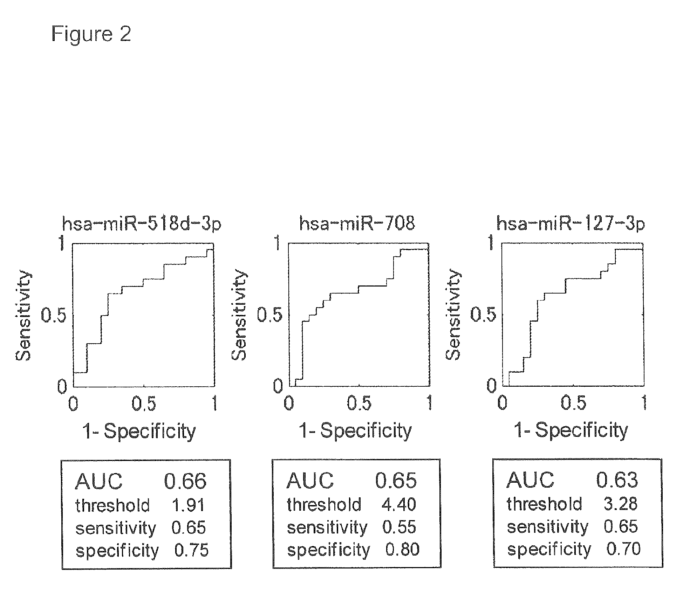

First, the detectability of each of the three types of miRNAs: hsa-miR-708, hsa-miR-127-3p, hsa-miR-518d-3p, when they were used singly, was compared based on the area under the curve of the Roc curve (AUC). Further, on the ROC curve, a point at which both groups can be most efficiently distinguished was identified. The cutoff value (threshold) corresponding to the point was regarded (expressed) as the upregulated control rate (Fold Change) based on the control-group average expression level. Next, specificity true positive rate (Sensitivity) and specificity true negative rate (Specificity) were calculated when the cutoff value was used. These are shown in Figure 2.

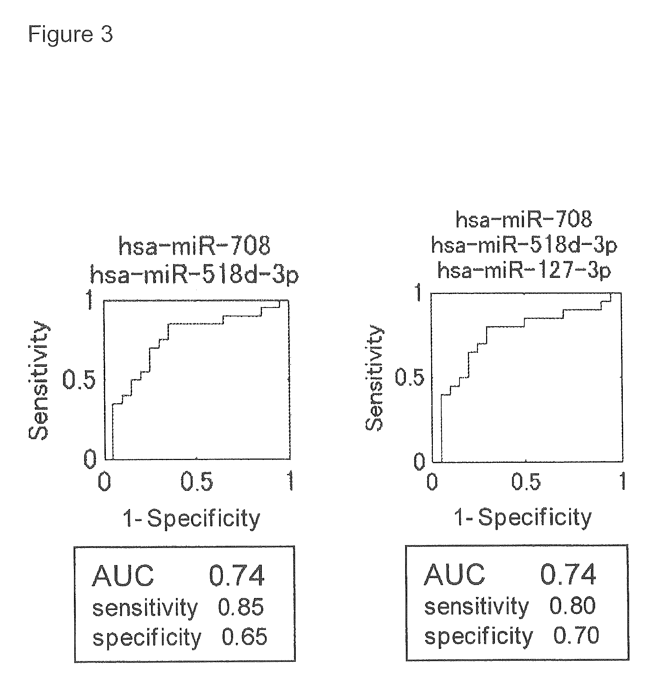

A prediction model was constructed by using plural miRNAs in combination based on logistic regression analysis and detectability of the model was evaluated by ROC analysis in the same manner as above. Figure 3 shows, a ROC curve (left) when a prediction model constructed of hsa-miR-708 and hsa-miR-518d-3p was used, or a ROC curve (right) when a prediction model constructed of all of hsa-miR-708, hsa-miR-127-3p and hsa-miR-518d-3p (right) was used. Detectability was evaluated based on the areas under the curves (AUC).

As a result, the area under the curve of the ROC curve when using a model of two or three miRNAs in combination was larger than when using a model of a single miRNA (Figure 2), demonstrating that a patient can be detected with a higher accuracy.

### Industrial Applicability

The present invention is useful for diagnosis of endometriosis.

## Claims

1. A diagnostic agent for endometriosis for measuring the concentration of at least one miRNA selected from the group consisting of hsa-miR-708, hsa-miR-127-3p and hsa-miR-518d-3p in a sample derived from the blood of a subject, the agent comprising an amplification primer for the miRNA as a main component.

2. A kit for diagnosing endometriosis comprising the diagnostic agent according to Claim 1.

3. The diagnostic agent for endometriosis according to Claim 1, wherein the miRNA is hsa-miR-708.

4. The diagnostic agent for endometriosis according to Claim 1, wherein the miRNA is hsa-miR-127-3p.

5. The diagnostic agent for endometriosis according to Claim 1, wherein the miRNA is hsa-miR-518d-3p.

6. The diagnostic agent for endometriosis according to Claim 1, wherein the sample derived from the blood is blood plasma.

7. A method of detecting endometriosis, comprising a step of measuring the concentration of at least one miRNA selected from the group consisting of hsa-miR-708, hsa-miR-127-3p and hsa-miR-518d-3p in a sample derived from the blood of a subject.

8. The method according to Claim 7, comprising a step of determining whether the concentration of the miRNA is higher than the concentration of the miRNA in a normal control.

9. The method according to Claim 7, comprising a step of determining whether the concentration of the miRNA is a cutoff value or more.

10. The method according to Claim 7, wherein the miRNA is hsa-miR-708.

11. The method according to Claim 7, wherein the miRNA is hsa-miR-127-3p.

12. The method according to Claim 7, wherein the miRNA is hsa-miR-518d-3p.

13. The method according to Claim 7, wherein the sample derived from the blood is blood plasma.

14. A method for diagnosing endometriosis in a subject, comprising a step of measuring the concentration of at least one miRNA selected from the group consisting of hsa-miR-708, hsa-miR-127-3p and hsa-miR-518d-3p in a sample derived from the blood of the subject.

15. The method according to Claim 14, comprising a step of determining whether the concentration of the miRNA is higher than the concentration of the miRNA in a normal control.

16. The method according to Claim 14, comprising a step of determining whether the concentration of the miRNA is a cutoff value or more.
